(19) **Europäisches Patentamt / European Patent Office / Office européen des brevets**

(11) **EP 4 763 089 A1**

(12) **EUROPEAN PATENT APPLICATION**

(43) Date of publication:
**24.06.2026 Bulletin 2026/26**

(21) Application number: **24220768.6**

(22) Date of filing: **17.12.2024**

(51) International Patent Classification (IPC):
***A61B 6/00*** *(2024.01)*

(52) Cooperative Patent Classification (CPC):
**A61B 6/5241; A61B 6/465; A61B 6/54;** A61B 6/03;
A61B 6/4283; A61B 6/4417; A61B 6/4429;
A61B 6/4441; A61B 6/4452; A61B 6/469;
A61B 6/488; A61B 6/5247; A61B 6/547

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.
5656 AE Eindhoven (NL)**

(72) Inventors:
• **MAY, Jan Marek
Eindhoven (NL)**

• **LUNDT, Bernd
Eindhoven (NL)**
• **KOEPNICK, Johannes
5656AG Eindhoven (NL)**
• **SINGH, Nikitha G
Eindhoven (NL)**
• **JAISWAL, Ashish
Eindhoven (NL)**
• **PATIL, Abhijit
Eindhoven (NL)**

(74) Representative: **Philips Intellectual Property &
Standards
High Tech Campus 34
5656 AE Eindhoven (NL)**

(54) **X-RAY IMAGE STITCHING**

(57) A system (SF) and related method for facilitating obtaining sub-images ($m_j$) of a region of interest, ROI, (R) for combining into a target image (M) of the ROI (R). The ROI (R) may be situatable in an examination region (ER) of an X-ray imaging apparatus (IA). The system may receive, at an input interface (IN), input data including data ($d$) indicative of the ROI. The system computes, prior to X-ray exposure, based on the input data, a plurality of sub-FOVs, wherein the plurality of sub-FOVs in combination are capable to cover the ROI.

Fig. 4

EP 4 763 089 A1

**Description**

FIELD OF THE INVENTION

The invention relates to a method of facilitating imaging, to related system, to an imaging arrangement including such as system, to a computer program element, and to a computer readable medium.

BACKGROUND OF THE INVENTION

Image stitching is a common technique in X-ray imaging to create images of anatomies larger than an X-ray detector size available for acquisition. In some current approaches, overlapping X-ray sub-images of the target anatomy are acquired. Next, adjacent images are aligned and blended by an image processing algorithm to obtain one image of the entire target anatomy. Current image processing algorithms commonly estimate the expected translation between two adjacent sub-images by similarity measures, such as normalized cross-correlation, or the sum of absolute differences between the image intensity signals, or other such similarity metric. It has been found that such metrics are prone to (plural) local maxima or minima, which might be caused by noise or parallax errors. Such local extrema of such similarity metrics might result in inaccurate stitching results, or even failure of the entire stitching procedure.

Some current image stitching is based on such similarity metrics that are directly computed off overlapping X-ray sub-images, and the overlap between such sub-images can be sizable. A main disadvantage is that such overlapping area is exposed twice to X-ray radiation (or X-radiation in short as used herein), thus leading to a higher dose cost for the patient.

SUMMARY OF THE INVENTION

There may therefore be a need for improved imaging for regions of interest that exceed in size the available X-ray detector's effective size. In particular, there may be a need for improved image stitching approaches.

An object of the present invention is achieved by the subject matter of the independent claims where further embodiments are incorporated in the dependent claims. It should be noted that the following described aspect of the invention equally applies to the method, to the related system, to the computer program element, and to the computer readable medium.

According to a first aspect of the invention there is provided a system for facilitating obtaining sub-images of a region of interest ("ROI") for combining into a target image (M) of the ROI, the said ROI being situatable in an examination region of an X-ray imaging apparatus, wherein the system is to receive, at an input interface, input data including data indicative of the ROI, and the system capable of computing, prior to X-ray exposure, based on the input data, a plurality of sub-FOVs, wherein the plurality of sub-FOVs in combination are capable to cover the ROI.

In embodiments, the ROI relates to a given subject, such as a patient, to be imaged, but may also relate to other subject(s), animate or not.

The proposed system is of particular benefit if the X-ray imaging apparatus's X-ray detector has an effective size smaller than that of the ROI. For example, the ROI may relate to a larger sized anatomy, such as chest, femur, or any other, in relation to the size of the detector's effective size.

In embodiments, the system is capable of so computing based on a partitioner , wherein partitioner is operable to compute, based on the input data, a partition of at least a portion of a planned projection domain in relation to the imaging apparatus into planned sub-field-of-views, FOV, ($v_j$) at which the sub-images are acquirable by the X-ray detector at different imaging geometries ($g_j$).

In embodiments, the system comprises an output interface for passing on a specification of the partition to control circuitry of the imaging apparatus for processing and based on the processing, to causing the imaging apparatus (IA) to acquire the sub-images at the sub-FOVs .

In embodiments, the control circuitry includes, or capable of interfacing with, an imaging geometry determiner/-adjuster capable of determining/adjusting imaging geometry settings ( that enable the imaging apparatus to acquire said sub-imagery at the sub-FOVs .

In embodiments, the said imaging geometry includes in particular a pose of any or more of X-ay source and/or detector. Thus, orientation and/or position of at least one of X-ray source and X-ray detector may be adjusted. Other imaging geometry may include pose of subject support (eg, patient support) or settings of collimator, etc, and any other relevant imaging, or imaging supporting, component(s). Thus, imaging geometry determiner capable of determining imaging geometry settings and cause adjustment of the imaging geometry that enable the imaging for said sub-imagery at the sub-FOVs . During imaging, the planned projection domain turns into an actual (instantiated) projection domain, wherein, for example, pose of detector is adjusted and/or the X-ray, and/or pose or setting of any other of the said imaging, or imaging supporting, component(s).

The range of allowed imaging geometry may be suitable constrained so that the planned projection domain is confined to a surface in 3D space, such as plane ((planned) imaging plane). Suitably so constraining may speed up the search of the planned sub-FOVs, for example, and may make the system and related method more responsive overall.

In embodiments, the system comprises an image combiner capable of combining the sub-images, once acquired, into the target image .

In embodiments, there is at least one overlap of adjacent sub-FOVs.

In embodiments, the partition is definable in terms of location of the said at least one overlap in the planned projection domain , given an overlap size and/or size of the sub-FOVs.

In embodiments, the input data includes specification of any one or more of:

i) a respective size of the sub-FOVs,
ii) an indication of a size of the, or a, at least one overlap of any adjacent sub-FOVs, and
iii) spatial data indicative of spatial distribution of any one of more of a) local radiation sensitivity of the ROI, and b) local structural data within the ROI.

The respective size may be preset as design parameter or as user adjustable variable to define solution space over which the system optimizes.

In embodiments, the said structural data includes any one or more of self-similarity or variational data. The structure may pertain or spatial structure and/or physiological response to X-ray radiation. Such data may be based on prior (already extant) imagery from a suitable imaging modality (which or may not be different from X-ray), to anatomical/physiological model, atlas, etc, or any other.

In embodiments, the ROI relates to a given subject, such as a patient, to be imaged.

In embodiments, the structural data is spatial data. It may be in form of a respective map or other scalar field defined (via registration or natively), on a grid of points in the planned projection domain.

In embodiments the spatial data is a self-similarity map of reference human anatomy, such as an anatomical atlas or similar.

In embodiments the self-similarity map describes the self-similarity between neighboring regions in prior data, such as in prior X-ray images drawn from the same patient, or from one or more different patients (population of patients) as may be found in PACS or other image storage. Imagery of other modalities such as CT, MRI or nuclear may be used instead as prior data.

In embodiments the self-similarity map may be generated from prior full body or stitched X-ray images of the same or other patients.

In embodiments the self-similarity map may be generated from 2D forward-projections of 3D CT volume data. Using such tomographic image domain data or other 3D data allows defining the self-similarity map for the ROI from any of multiple projection views.

In embodiments, the radiation sensitivity map may pertain to anatomical information from a population of patients, from a radiology atlas or other. The sensitivity map indicates per region radiation sensitivity for human tissue. This allows steering the overlap away from a region for which radiation exposure should be minimal, e.g., reproductive organs, etc.

In embodiments, the radiation sensitivity map may be generated by expert annotation.

In embodiments, the radiation sensitivity map may also be generated based on a deformable 3D model of reference human body. This enables distinctions in sensitivity depending on the viewing angle.

In embodiments operation of the partitioner is driven by an objective function in an optimization setup. For example, the said operation may be driven so as to find, eg in an iterative manner, solution(s) in solution space (the space of some or all sub-FOV definitions) that improve, eg optimize, objective function. The objective function may be a cost or utility function, and the said improving pertains to reducing cost and increasing utility, respectively.

In embodiments the objective function is configured to respond to any one or both of: a) distribution of local radiation sensitivities of the ROI, and b) distribution of local structural data within the ROI.

In embodiments, the spatial data is based on non-ionizing measurements in relation to the ROI, the measurements acquirable by a sensor.

In embodiments, the said sensor includes any one of more of: an optical camera, a depth sensing camera, LIDAR, a pressure sensor mat.

The sensor may be a camera configured for providing the non-ionizing measurements as an optical image, such as an RGB (color/grey value) image, or with a depth channel RGBD, or a depth image of a patient to be imaged.

In embodiments, the method may comprise registering the self-similarity map of the standard human body to the sensor measurements (e.g., RGB, RGBD or depth image, etc.) of patient to be imaged. In embodiments, the method may comprise in addition or instead, registering the sensitivity map of the standard human body to the RGB, RGBD or depth image of the human body to be imaged.

In embodiments, the spatial data is obtainable by operation of a spatial registry component operable to spatially the respective prior data (g,s) or prior model in relation to radiation sensitivity or structural data to the measurements.

In embodiments the system includes, supports or is capable of interfacing with a user interface through which at least a part of the input data is providable.

In another aspect the is provided a medical imaging arrangement, comprising at least a part of the system of any one of the preceding embodiments and aspect, and further comprising any one or more of: i) the imaging apparatus, ii) one or more memories on which is stored at least part of the input data, iii) the said sensor.

In another aspect there is provided a computer-implemented method of facilitating obtaining sub-images of a region of interest, ROI, for combining into a target image of the ROI, the said ROI being situatable in an examination region of an X-ray imaging apparatus, the method comprising:

receiving input data including:
data indictive of the ROI; and
computing, prior to X-ray exposure, based on the input data, a plurality of sub-FOVs, wherein the plurality of sub-FOVs in combination are capable to cover the ROI.

In embodiments, the said computing comprises computing a partition of at least a portion of a planned projection domain in relation to the imaging apparatus into planned sub-FOVs ,at which the sub-images are acquirable by the X-ray detector at different imaging geometries.

In an aspect, the method may comprise:

receiving:

- an X-ray image sub-FOV size;
- number of X-ray sub-FOVs required and
- a size of the overlap;
- optimizing (improving objective function) in respect of overlap between some or any two adjacent sub-FOVs, based on any one or both of the self-similarity map and the sensitivity map.

The size of the overlap may be function of self-similarity or variation. If the self-similarity is low, or variation is high, a smaller overlap can be used.

In another aspect there is provided a method of generating the said spatial or prior data, in particular for use in any one of the above methods and/or systems.

In another aspect there is provided a computer program element, which, when being executed by at least one processing system (eg, computing system), is adapted to cause the processing system to perform any one of the above methods.

In another aspect there is provided at least one computer readable medium having stored thereon the said program element.

Thus, what is proposed herein is an automated X-ray image stitching planning. It is based on using, in some embodiments, structural data, such as self-similarity maps and/or radiation sensitivity maps. The planning is operable pre-acquisition. By using an optimization approach based on prior spatial data, such as self-similarity map and radiation sensitivity map, the "dilemma" in the current image stitching art can be addressed, in particular for image stitching approaches that are based on optimization of an objective function, such as of a cost function. This dilemma arises as follows: as mentioned above, such current image stitching algorithms rely on overlapping sub-images in order to work. Hence, one may have thought that it may be desirable to keep the overlapping area as small as possible to safe dose. However, this is usually not possible because, as the overlapping area becomes smaller, the global minimum of the cost function gets less pronounced, and ambiguities can arise: the stitching can fail, or may incur artifacts. The proposed approach, whilst also based on overlaps, avoids this dilemma, by placing them in locations where radiation sensitivity of the respective tissue, organ, anatomy, etc. to radiation is low. This objective tends to "nudge" the optimization towards solution(s) with smaller overlaps and at lower dose cost for sensitive anatomies if the radiation sensitivity map is used, in addition to the structural data.

Specifically, it is proposed herein in embodiments, to use self-similarity maps and/or radiation sensitivity maps in respect of the human body. Such can be derived for example from extant, earlier, X-ray of the same or a similar patient. Such prior spatial data can be registered to a current spatial measurement, such an RGBD image or other, acquired by camera or other sensor when patient is in the examination region of the imaging apparatus. The prior maps can be used to automatically calculate an optimal stitching partitioning, based on radiation sensitivity and stitching conditions. Hence, the stitching failure rate and the effective dose will be reduced as both criteria are accounted for in the proposed system and method which is preferably practiced in a numerical optimization setup.

Thus, the proposed setup may be based on prior contextual data, including the self-similarity map and the radiation sensitivity map. The self-similarity map represents structural self-similarity of refence anatomical spatial data, such as prior X-ray imagery, atlases, etc. Such a map may be defined as deviation from an average value in per sample neighborhood at a grid point/location, or a gradient map, or other. The radiation sensitivity map represents how sensitive a certain tissue or organ/anatomy is to X-radiation at a reference energy. For example, such map may assign a tissue weighting factor, for example, such as per the ICRP standard, 2007, the 2007 "Recommendations of the International Commission on Radiological Protection", ICRP Publication 103, Ann. ICRP vol 37 Nos 2-4, 2007., or equivalent or other. However, this standard is merely one example. Any other metrics or measure may be used to represent tissue response to X-radiation.

The structural self-similarity map in respect of the human body may be based on X-ray images. The map can be constructed based on a data set of full body X-rays e.g. acquired by existing stitching runs, or by forward projection through CT full body scans, etc. However, such prior full body imagery (X-ray, CT, MRI, *etc.)* is not necessarily needed, as long as the prior imagery covers a large enough area. For example, abdominal prior imagery, etc. may be sufficient, if this is where the intended ROI is located. The radiation sensitivity map may be constructed based on the available radiation sensitivity information for organs, tissue type, etc., in order to calculate the effective dose. Expert annotation of prior imagery may be used, similar to the imagery used to construct the similarity-map. A registration of the self-similarity map and/or pf the radiation sensitivity map may be done using current RGBD image or other spatial measurement of the patient at hand. An optimal selection of the partitioning into planned field of views (FOV) may be based on a weighted trade-off between effective dose and ideal stitching conditions given a user defined target range for the stitching run.

The proposed system and method implement image stitching planning method. It provides imaging geometry settings for acquiring projection sub-images (frames) capable of tiling an intended ROI at overlaps. The system and method provide a partition of (at least a portion) of the planned projection domain into intended/planned sub-field-of-views for the sub-images, at least one such sub-FOV per intended sub-image. Then, once the sub-FOVs are defined, the sub-image acquisition can commence. This may include repeatedly adjusting imaging geometry to acquire the sub-images at the respective sub-FOV. The locations of the overlaps are chosen at places where there is a good trade-off between radiation sensitivity and structural self-similarity. According to the proposed approach, sub-images may be taken with reduced exposure to X-radiation of sensitive organs, while allowing for improved stitching performance when considering self-similarity.

The proposed system is applicable in any X-ray systems in radiography or fluoroscopy, in particular those that have a camera system or other suitable sensor system in place, such as RGB with a depth channel RGBD, or a depth sensor without optical image, etc.

The following definitions are used throughout this specification.

*"user"* relates to a person, such as medical personnel or other, operating the imaging apparatus or overseeing the imaging procedure. In other words, the user is in general not the subject of the imaging (such as a patient).

*"optimization"* as used herein includes method and algorithm from numerical analysis that are based on an objective function (such as cost or utility function). The function is generally real-valued, positive. It maps solution variables in solution space into the reals. In optimization one attempts to find a solution by improving the cost function, in one or more iterations, over possibly multiple intermediate results, to find the final, the "optimal", result. However, the use of the term "optimization" does not mean that necessarily a global optimum is found. Local optima may suffice. Equally, a good many optimization setups are iterative, optimization herein does not necessarily require full and complete convergence. At times, the iterations are aborted once a user defined stopping condition is met. The intermediate solution thus obtained may be output as the "optimal" solution.

The term *"subject"* as used herein is to refer to a human or animal patient, but may also refer instead to an inanimate subject. To the extent that the below refers to "patient", this should be construed as example reference, as a synecdochic reference, with the understanding that the below equally pertains to any other subject to be imaged.

The term *"effective"* detector size may refer to the native full physical size (area or a length) of the radiation sensitive area/surface of an X-ray detector, or may relate to a smaller size, a portion of the native size, that is available for imaging, for whatever reason. For example, this may be for avoiding artifacts caused by a fan beam at border portion of the native radiation sensitive area/surface. This reduced smaller size may be brought about by collimation or in whichever way.

*"structural data"* or similar may be used herein as a term to refer to data definable in terms of a scalar field ("maps") that describe local structure in relation to the subject to be imaged. Structural data may include herein self-similarity map or variation map, or any other related data. Areas with low self-similarity or high variation are preferred herein. Variation data may be described based on gradient behaviour, or any other suitable manner.

The term *"cover"* as used herein in relation to the planned sub-FOVs and a projection view of the ROI refers to the situation where the ROI is a subset of the union of some or all sub-FOVs. Thus, the sub-FOVs are preferably to fully cover substantially the whole of the ROI.

BRIEF DESCRIPTION OF THE DRAWINGS

Exemplary embodiments of the invention will now be described with reference to the following drawings, which, unless stated otherwise, are not to scale, wherein:

Fig. 1 shows a schematic block diagram of an imaging arrangement;
Fig. 2 illustrates a partitioning into planned field of views of an imaging surface of an imaging apparatus;
Figs. 3A illustrates spatial data as may be used in the partitioning for the purpose of sub-image acquisition;
Fig. 3B illustrates a partitioning operation of an imaging surface of an imaging apparatus;
Fig. 4 shows a block diagram of components of a facilitator system as may be used in the facilitation of acquiring sub-images for combination into a super image of a region of interest that is larger than a detector of an imaging apparatus to be used for said acquisition;
Fig. 5 shows an illustration of objective functions as may be used in optimizations for the above mentioned partitioning; and
Fig. 6 shows a flow chart of a method of facilitating acquiring sub-images for combination into a super-image of a region of interest that is larger than the X-ray detector of an imaging apparatus to be used for the acquisition.

## DETAILED DESCRIPTION OF EMBODIMENTS

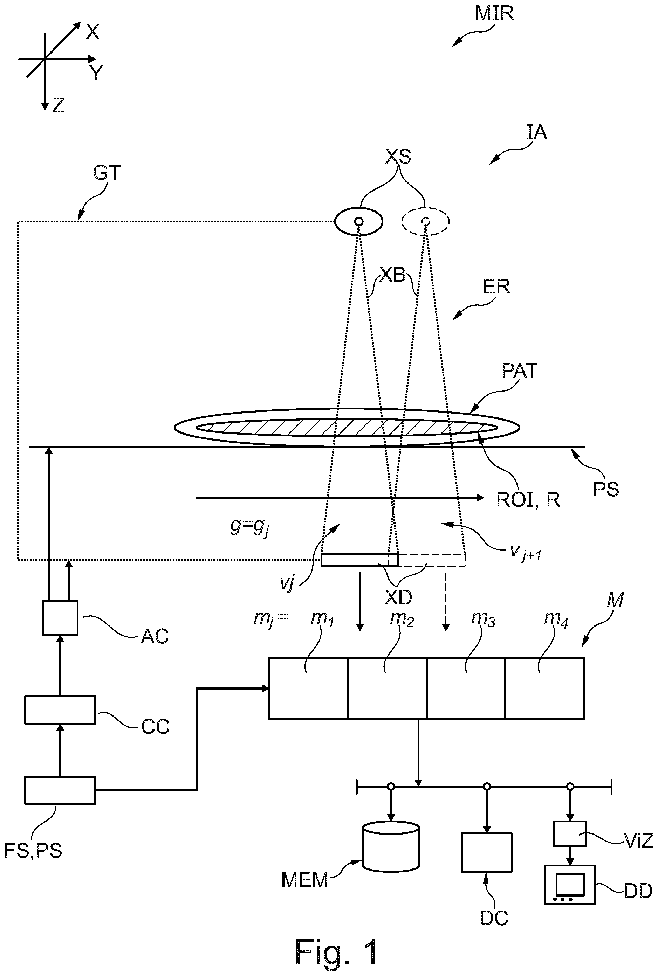

With reference to Fig. 1, this shows a schematic block diagram of an imaging arrangement MIR, preferably envisaged herein for medical applications, but others are not excluded. However, the following, main reference will be made to medical realm, thus, referring herein to such a medical imaging arrangement MIR, and it to include an imaging apparatus IA ("imager" IA for short), preferably of the medial type. More specifically, the imaging apparatus IA is preferably of the X-ray type. Thus, it uses X-radiation XB, producible by an X-ray source XS of imager IA, to acquire an image M of a region of interest, such as anatomy, organ or tissue of a patient PAT. The so acquired imagery M is projection imagery. Thus, it confers projection view across the patient, and this is recorded by X-ray detector XD of the imaging apparatus IA. The imagery M may be used for therapy or diagnosis or can be used for planning or any other purposes medical or indeed non-medical such as in non-destructive material testing or other application as need be.

The imagery M so acquired may be stored in a data memory MEM and/or may be consumed by visualizer VIZ and/or other data consumer DC. Visualizer VIZ may be operable to cause visualizing the imagery M on a display device DD, to so support a medical in formulating a strategy for diagnosis, treatment, planning etc., based on the visualized/displayed image. In addition, or instead, other data consumer DC may process the imagery in any way suitable and conducive to the task at hand. For example, data consumer DC may include an artificial intelligence diagnostic aid, or any other.

Broadly, and as will be developed in more detail below, the imaging arrangement MIR may include a specially configured, novel, computing system, referred to herein as the facilitator system FS (or facilitator FS for short). Such facilitator FS is operable to facilitate acquiring imagery M of the whole of a projection view (also referred to herein as "projection footprint") of the region of interest, despite the detector XD having an effective size (simply referred hereinbelow as the "detector size") smaller (in area or a length, than an area or a length of the said projection footprint of the region of interest ROI, R. This is achievable herein by facilitator FS orchestrating the acquiring, at first instance, of sub-images $mj := \{mj\}$. The Fig. illustrates an example for four such sub-images m1-m4, but there may be more or less such sub-images that are to be combined into the said image M, which, may be thus understood as a "super-image". Super-image, or target image as this is the actual goal of the imaging, confers a panoramic view of the whole of the projection footprint of the region of interest ROI, which, as said, may be larger than the actual image radiation sensitive surface of the X-ray detector XD. Thus, none of the sub-images, on its own, is capable of covering the whole of footprint $\pi(ROI)$ of the region of interest ROI, but in combination they do. This combination, as an operation, is sometimes referred to as image stitching. The facilitator system FS is operable to help control operation of the imaging apparatus to acquire suitable such sub-imagery at the "right" locations (in a sense to be made more precise below), which that can be so combined into the super-image or target image $M$.

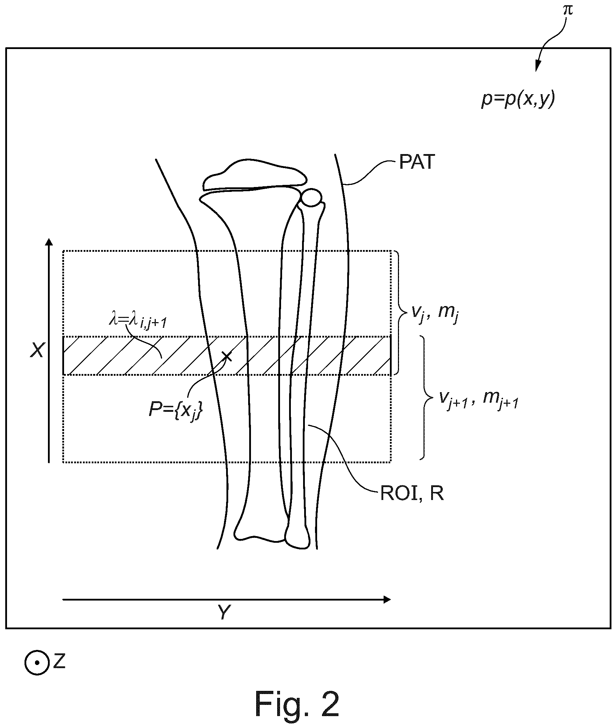

Preferably, and advantageously, the facilitator system FS is operable in a planning stage, pre-acquisition, that is, prior to exposure of the region of interest, or of the patient more generally, to X-radiation produced by the said X-ray source XS of the imaging apparatus. Prior to such acquisition of such sub-imagery $mj$, and as will be explained in more detail below, the facilitator system FS is operable to compute spatial data P (illustrated in Fig. 2). Based on such spatial data P, imaging geometry $g$ may be adjusting in respect of each of the sub-images $mj$ to be acquired in a particular manner to ensure efficient imaging stitching later on in a dose conservative manner. Once the imaging geometries are adjusted based on data P, the respective sub-images mj are then actually acquired in turns, and these can then be stitched together, or otherwise combined, into the target image M. The facilitator FS itself may include plural functionalities, parts of which are operable in the said planning stage preacquisition (that is, prior to any X-ray exposure), and one or more other such functionalities may be operable post-acquisition, in particular in stitching or combining the sub-images $mj$ into the wanted super-image $M$. However, the stitching or combining functionality may not necessarily be part of the facilitator's system as such. It can be instead commissioned from, and thus out-sourced to, other one or more computational agents, located elsewhere in a data communication network, possibly remotely from the facilitator FS, as needed.

Broadly, but in more detail, the facilitator system FS processes input data D, which will be described in more detail below, to produce the said spatial data P. The spatial data P is used by acquisition control circuitry CC in effecting the acquisition of the sub-images *mj.* The control circuitry CC may interface with one or more actuators AC to effect changing or adjusting the imaging geometry for each respective sub-image, and then cause acquiring same by sending a trigger signal to the X-ray source to trigger radiation exposure at the adjusted imaging geometry. Thus, the control circuitry CC may be operable to effect a number of different imaging geometries, one for each of the sub-images, and these are then acquired in any suitable sequence until all sub-images are collected.

Broadly, in operation, subject, such as a patient PAT, either sits, stands, or otherwise resides in an examination region ER of the imaging apparatus IA. The said examination region ER is a portion of the 3D space between the X-ray source XS and the X-ray detector XD. During acquisition, the X-ray source XS is energized by the control circuitry CC, either automatically or under direction of an operator control console by the user, such as a technician. X-ray beam XB issues forth from a focal spot (not shown) of the X-ray source XS, passes through patient tissue, in particular through parts of the region of interest, and is then incident on a radiation sensitive surface of the X-ray detector XD to be detected there as spatially resolved set of different intensity measurements. This is because during the passage of the X-ray beam XB through the patient the radiation is modified, such as by attenuation, scattering or other matter vs radiation interaction effects. It is then the so modified radiation that is then detected as said intensity measurements at the X-ray detector. The patient may reside on a patient support PS, such as a couch, table, etc., but such is not necessarily needed herein. For example, in chest X-rays the patient may well stand or sit in the examination region during the acquisition, rather than lying.

An intended projection view of interest on the ROI, R may be larger (in area size) than the radiation sensitive surface of the detector XD. Thus, along certain spatial dimensions, the region ROI may exceed a length taken over the radiation sensitive surface of the detector XD. For example, on occasion, the region of interest ROI, R may include patient's chest, which for large individual my measure an area 50 x 50 cm, whilst the X-ray detector XD's imaging surface may be much smaller, at 20x20cm or so. Similar problems may arise when imaging large bones, such as the human femur, or any other anatomy, the spine. Again, the X-ray detector may be too small to allow capturing, in a single image, the whole of the projection view of the ROI, R. To this end, such smaller than ROI-size detector may still be used to capture the whole of the ROI, but int multiple exposures, with imaging geometry adjustments in between, to so acquire in sequence set of (two or more) sub-images that then together cover the large region of interest ROI. X-ray detector equipment tends to be expensive, and this scales further with the size of the detector. There is thus an incentive for smaller detector sizes, which, however, may not be capable of capturing the ROI in whole. The proposed imager IA is cost efficient as it allows, despite its smaller sized detector, to still image ROIs whose projection footprint outsizes the sensitive area of the detector.

In operation, the X-ray source XS, such as an X-ray tube, is energized by application of a current and voltage. Depending on the imager IA's type, the detector XD and the source XS may be arranged mechanically in a gantry GT, such as may be the case for imagers IA of the C or U-arm type, frequently used for interventional imaging, also envisaged herein. Such interventional imager IA allows re-adjusting the imaging geometries by moving the gantry GT, and with it source XS and detector XD (e.g., to a new position as shown in dashed lines in Fig. 1). Whichever X-ray imager type is used, the imaging geometry in general relates to the spatial 3D constellation or configuration of three parts, the X-ray source XS, the X-ray detector XD, and at least a part the ROI R. Broadly, any given imaging geometry may be defined by the location and/or orientation of an imaginary line that may be run from the focal spot the X-ray source to the center point of the X-radiation sensitive surface of the detector XD. The direction from the X-ray source to the X-ray detector may also be understood as propagation direction of a central ray of the X-ray beam issuing forth from the X-ray source's focal spot during imaging, that is, image acquisition. The X-ray source and/or the X-ray detector XD may have up to 6 degrees of spatial freedom each, such as one, any two, or all of roll, pitch and roll, around three perpendicular spatial axes *X, Y* and *Z*, and/or translations along any one, two, or all of the spatial axes *X, Y, Z*. The said axes span a coordinate system *(X, Y, Z)* which may be referred to herein as the imaging co-ordinate system. The axes *X, Y, Z* roughly correspond to the 2 spatial dimensions of detector's radiation sensitive surface, usually a plane, whilst direction *Z* is perpendicular thereto, and refers to the propagation direction along the said imaginary line mentioned earlier. Detectors of the flat panel are envisaged herein having such a 2D planar radiation sensitive surface, but detectors with having a curved radiation sensitive surface are not excluded herein. Preferably, the detector includes DAQ circuity for native digital image acquisition, but analogue type detectors including X-ray sensitive film encapsulated in a cassette are not excluded herein. Acquisitions may be post-digitized later, if needed.

Imagers IA of the mentioned interventional type, such as the C-arm systems or similar, may be used for cardiac imaging, such as for image guided procedures (e.g., fluoroscopic protocols). Such procedures may include placing a stent in an occluded vessel, to name just one example. However, such interventional imaging with that many degrees of freedom are not necessarily needed herein in some radiographic type imagers. Indeed, in some such radiographic imaging equipment as may be used for chest X-rays or orthopedic applications (trauma center), there may not even be a mechanical gantry at all that connects physically the X-ray source and the X-ray detector. For example, in some modern design radiography systems, the gantry is "distributed". For example, the X-ray detector may be wall, floor or ceiling mounted in its own gantry, whilst the X-ray detector is arranged in its own gantry, across the examination region ER at a distance therefrom in another part of the exam room. Such detector or source gantries may be arranged respectively as an articulated arm, a tracked system, or any other to allow positioning of the X-ray source or detector by shifting along two spatial dimensions, and/or by changing its orientation via suitable articulation conferring mechanical fixture, e.g., ball joint or the like.

As said, the X-ray detector XD may be a flat panel detector although in other embodiments the radiation sensitive imaging surface is curved for better dose efficiency, in particular when anti-scattering-grids are used. In examples, such as in the chest X-ray, the patient usually stands in the exam room, with the X-ray detector XD held in position and orientation behind patient at the right height aligned with back of patient's torso, whilst the source is positioned on the other side of the patient in front so that the X-ray beam is incident on the X-ray detector after passage through patient's torso.

The imaging geometry may be adjusted manually by manual actuators, such as hand wheels, levers, etc., or may be adjusted mechanically, or semi-automatically, by a servo- or stepper motors, or any other electro-mechanical single or plural actuator AC. In order to adjust/change imaging geometry, interfacing of actuator AC - there may be more than one, at least one for the source XS and/or at least one for the detector XD - may receive a control command from control circuitry CC, with such control circuitry itself receiving as input data computed by the proposed facilitator FS. The actuator AC may be interfaced with, or may include one or more positional/angular encoders for precise imaging geometry adjustments that are trackable by control system CC. Thus, whilst the output spatial data provided by facilitator FS may be processable by the control circuitry into lower-level control signals in order to drive actuator(s) AC automatically, in other embodiments the spatial output data provided by the facilitator FS is graphically rendered by visualizer VIZ or other such functionality in a graphics display. For example, the graphics display may be displayable on display device DD or other display device. Graphical user interface (GUI) may be so displayed. The data so rendered preferably includes dynamically updated instructional widget(s), capable of indicating visually directional or other progression, such as arrows, rollers, spinners, dials, etc. Such widgetry may be displayed in association with various components, such as the source and/or detector or related manual actuators AC, thus allowing users themselves to make the adjustments, under graphical guidance of the graphics display. The widgetry is updated in response to the user operating the actuator(s) AC when adjusting the imaging geometry. The widgetry is instructional in that it is capable of indicating visually to user the current imaging geometry, and the target imaging geometry as computed by facilitator FS. For example, the widgetry may be configured to visually indicate how the imaging geometry needs to be adjusted for acquisition of the first or next frame. For example, an interactive, visually dynamically updated arrow widget may indicate that source XS and/or detector XD may need to be shifted by *10cm* to the left, etc.

Referring now in detail to Fig. 2, this affords a projection view along the imaging axis Z, perpendicular to the two 2D imaging surface (in this case, a plane) spanned by spatial axis $X$ and $Y$. This imaging surface may be referred to herein as the planned projection domain $\pi$. This planned projection domain becomes the projection domain after planning, once detector is used to acquire the sub-images $mj$ as will described herein in more detail. As said, the projection domain is a plane, will be referred herein as such in the main, with curved projection domains not excluded herein. The 2D planned projection domain is the part of 3D space in which the acquired imagery is located. Planar or panel stitching is mainly envisaged herein, although more complex stitching setups over curved domain $\pi$ is not excluded herein as may be necessitated by a curved detector shape. Due to the planar imaging setup mainly envisaged herein, cartesian imaging domain co-ordinate system *(X,Y)* can be used. In a curved setup, a different coordinate system may be used that reflects the curved geometry, such as cylindrical coordinate system, or other. However, for the sake of illustration of principles as envisaged herein, the following will be confined in, the main, to simpler planar parallel stitching as shown by the example illustrated in Fig. 2.

One of the spatial axes of the planned imaging plane (planned projection domain) $\pi$, may run parallel to the longitudinal axis of the patient, which, in this example, without loss of generality, is taken to be axis X. Each imaging geometry $gj$ can be identified by the associated planned sub field of view $vj$. When the imaging geometry so adjusted, upon X-ray exposure from source XS, the actual sub-image $mj$ at that planned field of view vj is acquired. During imaging, the planned field of view becomes the actual field of view of the imager IA. As there are multiple sub-images $mj$ needed to cover ROI R, there are associated multiple such planned FOVs $vj$. The facilitator FS is operable to compute the said planned sub-FOVs $vj$. During operation of facilitator FS in the planning stage, no actual imaging geometry adjustment is needed at this time, but can still be done in parallel, if needed for better responsiveness, as the computed planned sub-FOVs vj become available. Rather, at this, the planning stage, facilitator FS merely provides spatial definitions of the planned sub-FOV *vj's*. Later, in acquisition, the planned sub-FOV or "partial FOV's" *vj* are then spatially instantiated by actual imaging geometry adjustment(s) *gj*. And the associated imagery *mj,* one or more per each such sub-FOV vj is then acquired. For illustration, two such planned sub-FOVs for respective different image geometry setting *gj* are illustrated in Fig. 2. There may well be more than two such sub-FOV *vj's* in general, depending on the size ratio between detector XD and the size of the intended projection view of ROI R. In some embodiments, the adjustment in imaging geometry amounts to changing pose (position and/or orientation) of the X-ray source XS and/or of the X-ray detector XD (see for instance two possible positions of XS and XD in Fig. 1). Thus, in some examples, some or each sub-image is acquired with the X-ray source and X-ray detector at different positions with respect to the subject PAT.

The planned image/imaging plane (planned projection domain) $\pi$ is conceptually made up of spatial grid points $p$ with co-ordinates $(x,y)$. One such point $p$ with its spatial co-ordinates $(x,y)$ is shown, indicating its grid position in $\pi$. With each change of imaging geometry $gj$, a different planned field of view $vj$ emerges, for example, one shifted and/or rotated relative to the other, and so on. For flat panel detectors, the planned sub-FOVs vj are quadrangular, and so are the sub-images, once acquired. This is shown in Fig. 2 with two fields of view vj, vj+1, one shifted along, for example, $X$ axis with respect to each other, each when used will allow acquiring X-ray sub-image $mj$, or $mj$ +1 respectively each having a different field of view. And the totality of such sub-images MJ acquired each at their different field of views $vj$ may then form the sought super-image M covering the whole of the region of interest in this case a bone as illustrated in Fig. 2. Thus, there is an association between position and/or orientation of the various field of views, and their associated imaging geometry settings $gj$ that when adjusted for, result in the respective field of view and can be used to acquire a sub-image at the respective field of view, once this geometry $gj$ has been assumed /adjusted for. Such adjusting may be caused by operation of the actuators AC as said earlier above, by re-positioning or re-orienting X-ray source XS and/or X-ray detector XD, as needed and depending on the imager IA type used.

The sub-images once acquired are "tiles" as they tile the ROI R. In the planning stage, prior acquisition, such tilling also applies to their respective planned field of views vj, which, at this planning stage, are simply sub-sets (quadrangles) of the planned projection domain $\pi$. Just like the later acquired sub-imagery, pairs of the planned FOV vj may be overlapping to form an overlap region $\lambda$ generically. Thus, there are different such overlapping regions $\lambda j,_{j+1}$ in respect of a pair of some or each adjacent views $vj, v_{j+1}$ as shown. Such overlapping region may also be referred to herein simply as "overlap". Such one or more overlap is desirable in order to produce robust stitching results. Thus, in the pre-acquisition planning stage, the facilitator FS is broadly operable to break-up or partition a part of the planned projection domain $\pi$. The partition is defined by the said spatial data P. Thus, the spatial data is, or defines, a particular partition $P$. Thus, it is apt herein to use the same notational symbol $P$ for the partition itself. The spatial data that is the partition P defines the spatial positions and orientation in the plane $\pi$ of the planned field of views $vj$'s that ought to be used in order to acquire images $mj$ that then suitably tile the whole region of interest. The facilitator FS is configured herein to find a particularly useful partition P* from possible partitions of the planned projection domain $\pi$. The facilitator FS can be conceptualized as a search facility that searches the solution space $\mathbf{P}(\pi)$ of possible partitions $P$ to so find a desirable one $P*$. The search is subject to certain constraints, as will be described in more detail below.

The partitioning itself may be spatially restricted and only applies to the portion of the imaging plane where the whole of the region of interest R resides. This rough localization can be provided by the user or may be determined automatically. Such localization may form part of input data as received by facilitator FS. In a user interactive embodiment, a graphical user interface may be supported, with a graphics display that represents the planned projection domain. As such, the graphics display may be similar in appearance to what Fig. 2 represents, optionally with a graphical depiction that represents the spatial extent of the region or of the patient as residing in the examination region. The graphics display may be obtained from photographic data (on which more below) suitably scaled to the currently displayed planned projection domain. The users can then indicate by drawing a bounding box or other, the portion that they wish to have partitioned. Thus, in whichever way defined, the said localization defines a range, a subset, of the imaging plane which ought to be partitioned into the planned field of views $vj$ in order to acquire the sub-images $mj$. To be sure, the planned projection domain is not as such the imaging/radiation sensitive surface of the detector XD. Rather, the planned projection domain is the surface in 3D space that is formed by the set of all loci that the detector can assume with suitably adjusted imaging geometry. Thus, this is the total surface as may be thought to be swept out by the detector XD when adjusting imaging geometry, which the panned projection domain is realized into the actual projection domain. The above described manners of defining the relevant portion of the planned projection domain whether by interactively by a GUI, by keyboard entries or in whichever way may be provided as an input functionality by the facilitator FS, or by any other computing entity as a kind of input data preprocessing or provisioning.

Each planned field of view vj may be defined spatially by equations or co-ordinates $P$ in the image plane $\pi$, or in any other suitable manner, each envisaged herein. For example, as shown in Fig. 2, each such planned FOV $vj$ may be defined by a reference to one or more points of the overlap that any two such adjacent planned FOVs vj, vj+1 are to form. For example, the collection of all respective mid-points $xj$ (illustrated as a cross-hair symbol $"x"$ in the Figure) of the respective intended overlaps between any two planned field of views $vj, vj+1$ may be used as an indicator of the said partition.

There are at least two planned field of views vj, with at least one overlap. In some cases there are multiple such planned FOV's $vj$, such as more than two FOVs, as there are more than two sub-images $mj$ needed to cover the whole of the region of interest R. Thus, there may be more than two overlap regions $\lambda$, that is, at least one for any two adjacent field of views $vj, vj+1$. The partitioning proceeds linearly only along one spatial dimension, such as along axis X as shown in Fig. 2. In this case, there is partitioning as per partition data $P=\{xj\}$ along a single axis, such as axis X as illustrated. Partitioning may proceed instead in the other direction $Y$ as needed or may proceed in a matrix fashion in 2D along both spatial axes, to form a tiling in multiple rows and columns, depending on convention, as needed. In this matrix type tiling, there may be overlaps not only in one direction but also in the other or in some cases there may be no overlaps in one direction such as $Y$ but there may be overlaps in the other direction X, as needed and depending ultimately on the size ratio between detector XD vs ROI R.

The one or more overlaps are currently used for "guidance" function for the stitching, but should ideally be kept as small as possible, in order to avoid unnecessary double exposure to X-radiation which can cause changes in cells' DNA which in turn can lead to cancer further down the line.

The partition P may be given by the set of all mid-points or other reference points {xj} of the respective overlaps $\lambda$ as indicated schematically in the Figure. Thus, the facilitator FS takes as input a range in the plane $\pi$ that needs partitioning, and outputs the said partition P in whichever forms, such as a set of co-ordinates, one or more geometric loci equations, as is suitable and convenient in the circumstances at hand. Such partitioning P may be in terms of any suitable reference point for each of the expected planned overlaps $\lambda$, such as their mid-point as shown, or any other, such as a corner point, etc. However, the partitioning is not necessarily defined by the location and size of the overlap. As the shape and size of the planned FOVs vj are usually known and set upfront as fixed system parameters, the partitioning may be defined instead in terms of position in image domain, such as by one or more suitable reference points, such as center point or corner point of planned FOV. In embodiments mainly envisaged herein, the planned FOVs are the same in shape and size among each other, such as the quadrangles shown in the Fig 1. That being said, partitioning into planned FOVs of different shapes and/or sizes are not excluded herein.

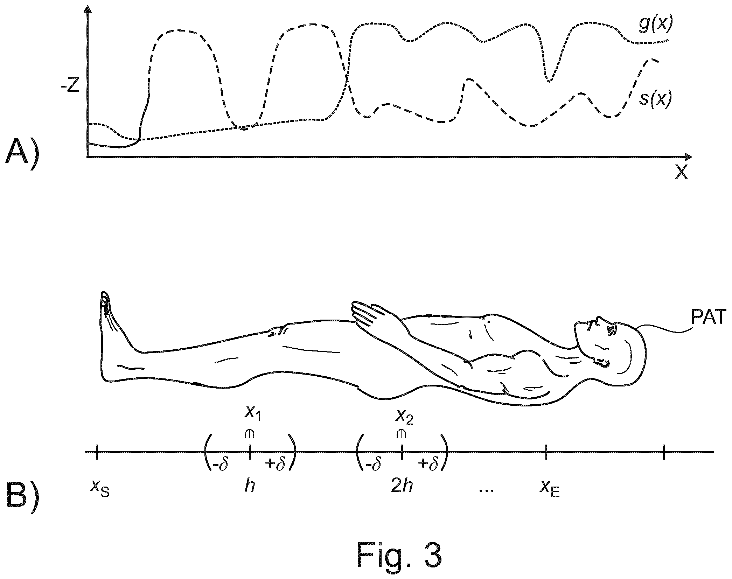

As mentioned, the facilitator FS is chiefly operable pre-acquisition, that is, before any image of any sort is actually obtained, to partition the relevant portion of the image plane $\pi$. The necessary structural data may thus be imported from external sources or data sets. Such external, or context data, is spatial data and is mapped to grid points $p$ of the planned projection domain, thereby conferring spatial structural data which is then used to guide the partitioning operation of facilitator FS. Because the patient is present in the examination region, such mapping will also map, by extension, to respective portion of the patient that happens to reside at the respective grid point. Specifically, facilitator FS's operation is guided by additional contextual data as is now illustrated in Fig. 3A, to which reference is now made.

The contextual data is spatial data and may comprise components $g,s$. The contextual data being spatial data may be made available to facilitator FS as maps or scalar fields, defined in the image plane $\pi$, although, for ease of illustration, the view afforded in Fig. 3A is along only one of the spatial direction $X$, with the other, $Y$, being into the drawing plane of the Figure.

One such data component, say $s=s(x,y)$, of the contextual spatial data may indicate spatially resolved radiation sensitivity of a tissue, as can be expected to be found at a given grid position $(x,y)$. This information allows guiding the positioning of the planned FOVs, such as their overlaps, thus computing the partitioning. Another data component, say $g=g(x,y)$, measures expected image structure, such as self-similarity, to be expected at the respective position $(x,y)$. Again, only the X axis co-ordinate is shown, revealing the map g (and s) in cross sectional view along axis $X$. However, at this point, because no image of the actual patient to be imaged is available generally, a general physiological anatomical model may be used which may be suitably registered onto the plane $\pi$ of the examination region in which the current patient resides, and, by extension, onto the actual patient PAT to be imaged. Equally, the radiation sensitivity information map g may be obtained from prior medical knowledge data.

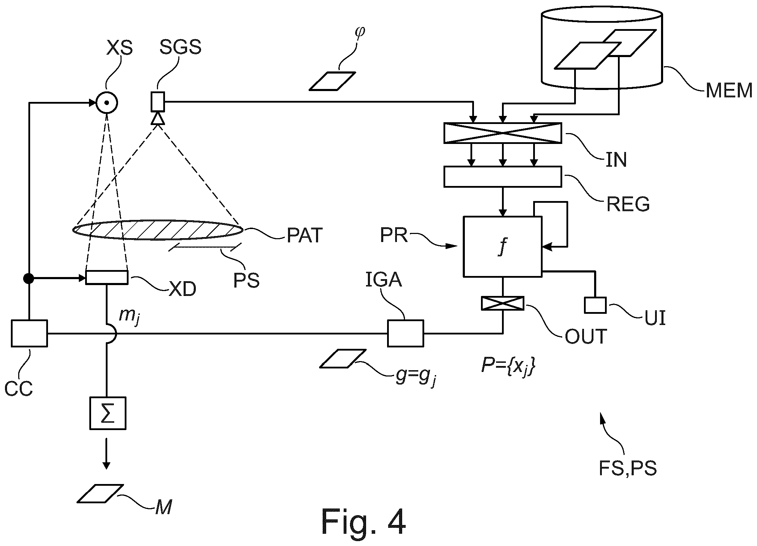

In some embodiments, the structural data may be obtained based on a very low dose surview scan (with dosage lower than the actual acquisition, later, of the sub-images). This may be done first by adjusting the imaging geometry to cover the area where the patient resides in one or more scans to so obtain spatial data, from which the above radiation sensitivity and image structure can be gotten by mapping onto. Whichever way, preferably the registration of the contextual data $g,s$ onto patient/ planned projection domain is gotten in a non-ionizing manner, such as by using an optical camera SGS with or without a depth channel, or any other sensor (e.g., pressure sensor mattress on which patient lies or sits, etc.). Such non-ionizing sensor SGS may be used herein in addition to the X-ray source XS. This sensor is used prior to the use of the X-ray source, such as in an exploratory operation, to obtain the spatially registered prior contextual data. The so spatially registered prior data $s,g$ is then used to guide the partitioning operation, as will be explained below with reference to Fig. 4. This figure shows in more detail functional components of the facilitator system FS, or components interfaceable with the facilitator FS.

Referring first and back to Fig. 3B, this illustrates operation of the facilitator system FS for an example, similar to the planar parallel stitching example of Fig. 2. The facilitator system FS receives input in terms of the initial range in the planned projection domain $\pi$ that covers the ROI R and which the user wishes to partition. In his 1D case (where partitioning is done only along one axis, say X) such range may be defined by an interval on X, between point XS and XE, Thus, only one spatial direction is shown. If 2D (matrix) partitioning is needed, the input data may include a similar spatial interval along the other spatial axis $Y$. Referring for illustration only and for the sake of simplicity to the 1D partitioning, this can be provided as at least one location $xj$ along $X$, thus in effect defining partitioning lines. In general, there will be more than one such location if there are more than two planned field of views vj needed to cover the whole of ROI R. Thus, the partition data as computed by facilitator is $P = \{xj\}$, the collection of all locations where edges of adjacent planned field of views are joined up. In some cases, a weighted sum of the pixels of the two images in the overlapping area may be used. If the stitching proceeds along the other spatial axis Y, $P = \{yj\}$, and, for 2D stitching, $P = \{(xj,yj)\}$. But again, the following will be confined mainly for the purposes of illustration to stitching along one spatial axis $X$ (the patient's longitudinal axis), with the understanding that the principles described herein are of equal application to the other axis Y, and to 2D stitching over the two axes *X, Y.*

The expected size of the individual planned FOVs vj (and hence of the sub-images) may be considered fixed, to lower the dimension of search space, thus increasing responsiveness of the system FS. In this case, the relevant size along *X* may be indicated by parameter *h.* This may be pre-set or may be computed by the facilitator system as will explained below in more detail. Thus, in embodiments search spaces of the *xj*'s of P are confined to steps at multiples of *h,* with width $\delta$ to either side of step *n\*h.* Thus, at each multiple (*n* =1, 2, ...), search interval (*nh-$\delta$, nh+$\delta$*) are defined in which the best partition xj is found. For example, in each such search interval, facilitator FS searches for the best placement of the mid points *xj* of the respective overlaps, subject to the constraints of data *g,s* at those intervals. In the example of Fig. 2, merely one overlap per pair of adjacent FOVs *vj,j+1* are shown, but this is for illustration only. There may be more than one overlap, such as two, one on either side of the joining line, or up to four, one for each side along *X* and *Y* in the 2D case. Because in each search interval (*nh-$\delta$, nh+$\delta$*), the distribution of radiation sensitivity and/or structural self-similarity as per *g,s* are in general different, the partition is a more complex pattern as each placement of *xj*'s may differ per interval. In general, for computational responsiveness, or for implementation on computational nodes with low power chipsets, size and/or shape of some or each *vj* is prescribed, in particular all *vj*'s have some space and/or size.

Referring now in more detail to Fig. 4, this shows the components of the facilitator system FS. It receives, at one or more of its input ports IN, spatial data $\varphi$ that relates to the patient to be imaged and how the patient position relates to the examination region, in particular to the planned projection domain. The facilitator FS draws in the contextual data *g,s* mentioned above in connection with radiation sensitivity and structural self-similarity, respectively. At this point, the spatial data, or maps, *g,s,* are based on reference data, such as anatomical atlas, model and the like. Thus, such standard data *g,s* may need to be personalized first to the actual patient PAT at hand. To this end, a spatial registration /interpolation component RG registers the patient's measurements $\varphi$ with the contextual data *g,s.* There may be different such registration /interpolation components RG for data g,s, respectively. The so personalized data may be provided up front. The standard data *g,s* prior to registration onto patient measurement $\varphi$ may be assembled from general medical, anatomic knowledge, models, atlases, etc. The patient measurement $\varphi$ may be provided by a non-ionizing sensor SGS, such as optical camera, depth sensing camera, LIDAR, etc. In addition, or instead, a low dose surview scan may be done instead. After such registration or "personalizes" by registration /interpolation component RG, the contextual data *g,s* map actual patient point positions *(x,y)* (points of patient in planned projection domain) to radiation sensitivity and self-similarity values. Thus, the spatial registration /interpolation component RG "ports" patient's anatomical structure into the otherwise abstract grid points of the planned projection domain. And it is this locally/spatially resolved structure (in terms of radiation sensitivity and self-similarity g,s) that is then used to guide the partitioning of the planned projection domain $\pi$.

The input data *d* received at input IN may further include range data. For example, the user may provide through a user interface IN the above-mentioned range $x_S$, $x_E$ of the portion of the domain that ought to be partitioned. Alternatively, a pre-processor processes the sensor SGS data and ascertains the applicable range automatically. This is then passed on, optionally on user approval, to the input port IN or processing.

A partitioner component PR then operates, based on an objective function *f* (which will be explored more fully below), to find the partition P in the indicated range. The said objective function *f* evaluates the personalized contextual data *g,s.* Once computational work by partitioner PR concludes, the partition data *P* is output at output port OUT. The partition data *P* may be provided for further processing. The partition data *P* represents overlapping planned FOVs *vj.* Specifically, the partition data *P* may represent position and orientation, etc., of the planned FOVs *vj* in the planned projection domain $\pi$. The partition data P may be in the form of co-ordinates in the planned projection domain $\pi$. The data P may relate to center points of the overlap regions $\lambda_{j,j+1}$ of adjacent *vj*'s., or may otherwise define the partition P, such as by corner points, e.g., left upper left, lower right etc., of the general quadrangular planned field of views vj, and the like.

The partition data *P* may be processed by an imaging geometry adjuster IGA into respective geometry settings *gj,* one for each planned FOV vj as per *P.* Each such *gj* is capable of "instantiating" the *vj* to which it relates: thus, the related geometry setting *gj* allows turning the respective planned FOV vj into an actual FOV.

The imaging geometry settings *gj* are processed by control circuitry CC to adjust the imaging geometry of imager to the respective field of view. Once adjusted, the control circuitry CC causes to energize the radiation source XS, thereby causing a respective X-ray exposure of patient PAT at each imaging geometry *gj,* Thus, at the related field of view *vj,* the respective sub-image *mj* is acquired. This is done for all settings gj, until the whole region of interest is covered (imaged by sub-images). The two or more sub-images *mj* may then be actually stitched together or otherwise combined by combiner $\Sigma$ into the super-image M. Any standard stitching algorithm may be used herein. Please note herein, that the super-image may not necessarily be displayed as a whole, but user may request only certain portions, such as by scrolling or similar. Thus, only a subset of the sub-images or the whole of the sub-images may be displayed at a time. The super-image may only exist in memory comprising the sub-images logically linked together, and these can be invoked by user when need to cause displaying of all or some of the sub-images. Not all sub-images may need to be stored in the same memory or storage. The sub-images may be stored in a distributed fashion across plural memories.

The above-described functionalities of facilitator FS are now explained in yet more detail.

Referring now first in more detail to the contextual spatial data *g,s,* that is, the self-similarity map and the radiation sensitivity map, one or both may be precomputed and later registered to the camera SGS data φ, such as depth image or other, prior to operation of the partitioner PR. Self-similarity in this context describes the similarity of an image area to the neighboring image areas, where an image can consist of an X-ray image and/or a depth map. The self-similarity map is computed based on prior images of the full human body and a self-similarity measure for every position along the vertical axis of the human body is obtained. However, such fill body data is not a necessity herein, as long as the map covers the whole region of interest. This measure represents the self-similarity at that specific location. Such prior images may be from other modalities, such as tomographic reconstructions, such as CT/MR/PET/SPECT, etc.

The radiation sensitivity map can be gained by projecting a segmented reconstruction of an earlier full body CT scan of the given patient, or a patient with similar biophysical characteristics, with annotated organ sensitivity applied by one or a group of radiology experts.

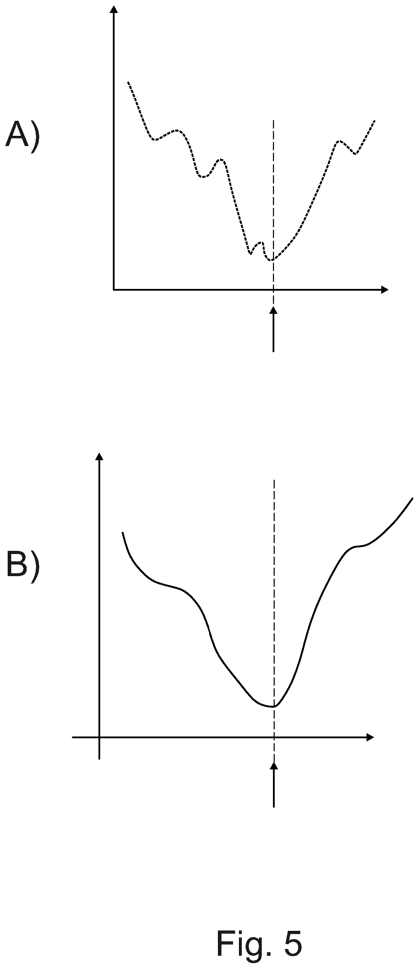

The maps *g,s* may be are registered to the current spatial measurement φ, such as an RGBd image, and this may be done when patient is in the examination room, using the (preferably deformable) reference map g and/or s, and the current depth image as per the depth channel ("D") of the acquired RGBD image. This allows the partitioner PR (see below at Eqs. (1-3) in more detail) to automatically find the placement of the overlap region $\lambda j,j+1$ between two planned FOVs *vj, vj+1* in a way that tends to minimize, in trade-off, the self-similarity, and the radiation sensitivity. This makes operation of the partitioner PR more robust. For example, in an optimization setup, driven by an objective function (e.g., cost function), this cost function is likely to have fewer or no ambiguities in where its local minimum is located, in respect of the best partition. In addition, a reduced overall dose is incurred as there is a tendency to keep the respective overlaps small (see further below, at Fig. 5, which is an illustration of such a cost function).

Referring now to the partitioner PR in more detail, this processing component may use an optimization algorithm. Thus, the proposed partitioning may be formulated as an optimization problem driven by an objective function *f*, such as minimizing or otherwise improving a cost function, and such formulation will be used herein, with the understanding that a dual formulation of maximization of a utility function can be done instead of asking for the minimum of the cost function as will be explained herein below.

In general, the cost function approach allows formulating the reconciliation or balancing of the two opposing needs for partitioning the planned projection domain, in particular with respect to the overlaps for neighboring field of views *vj j+1,* in 1D or 2D. The partitioning should be done so that overlaps are small in order to avoid unnecessary double exposure, but overlaps should be placed over regions of the domain π where there is sufficient image structure to allow a robustly "docking" of the later squired sub-images. Thus, the objective function is configured to foster obtaining a smooth super-image, with no or negligible transition artifacts between sub-images, at a low risk of radiation damage to tissue. Thus, the proposed setup allows clinical user to obtain, in a radiation efficient manner, a natural looking, higher fidelity panoramic image M of a large region of interest, larger than the detector that was used in the acquisition of the component sub-images *mj*.

The search space is the space of all partitions *P,* and the function *f* is defined over *P* as a possibly multi-dimensional real valued function. The cost function may be formulated as a sum comprising sub-cost functions, each responding to either the radiation sensitivity or the image structure self-similarity, respectively. A mix between two of these objectives can be achieved by forming linear combination with strength factors β,γ of *g,s,* as will be shown below. Considering both aspects radiation sensitivity *g* and image structure s together yields best results for present purposes, although considering only one (g or s), but not the other also envisaged herein in some embodiments. Thus, depending on the embodiments, contextual guiding data *g,s* may comprise either *g* or *s,* but not both. In embodiments, a suitable parametrization of partitions is used in a suitable solution space. For example, as mentioned, this may be done by using the mid-points xj of the respective overlaps, thus implicitly defining the positions of the respective field of views, given a pre-defined shape and/or size of the planned FOVs *vj.* The size and/or shape may be referred herein as the context parameters for the optimization, which are fixed, and are not part of the solution space. The user or a systems engineer may decide on the size (dimension) of the solution space by admitting into, or taking out, certain of the context parameters, such as FOV shape/size, as needed. A user interface UI may be used to adjust the parameters, by declaring them as fixed or as part of the solution space.

Thus, a higher dimensional solution space, and thus a higher dimensional parameterization, may be used, if needed, although for better responsiveness or for lowering demands on processor chip performance, the parameterization may be restricted. For example, a pre-defined height or width of the overlap may be pre-defined, or size/and or shape of the planned-FOVs may be predefined, such as parameter h,δ in the above example at Fig. 3B. The range of the overlap width in one or both spatial dimensions *X, Y,* may be predefined or are variables of the solution space. In the example of Fig. 3B, the shape of the overlaps, which in this case are formed of strips, is pre-defined, whereas the width of the strips is part of the solution variables over which the optimization is run. Thus, in this case, width and location within the respective δ-embodiments are solution variables, which, in embodiments, can be expressed as the said mid-points xj of those overlaps/strips. However, in more involved embodiments, higher dimension parameterization may be used in which the solution optimization also runs over the width h or other context parameters. Thus, in some embodiments, the planned FOV size(s) is/are not pre-defined but form one of the (solution) variables over which the optimization is run. The parameterization can be made higher dimensional still by also accounting for the number of tiles/planned-FOVs to be used, instead of this being pre-defined, for example, by user input UI. In general, the higher-dimensional the parameterization, that is, the higher-dimensional the solution space, the longer the algorithm may need to run or the higher the demand is in CPU performance, but with the added benefit that there are fewer upfront decisions asked off by the system FS. This may be of particular benefit to novice users, those will need less training or practical experience, or indeed for learners.

Specifically, the maximization problem solved by partitioner PR may be framed as:

$$P* = argmin_P f^u(P) \qquad (1)$$

Wherein $P*$ is the sought partition, and $u$ indicates contextual parameters that ought to remain fixed, so are outside solution space, whilst the $P$'s are inside solution space and form variables over which the optimization (1) is run.

Cost function $f$ may be based on the contextual objectives $g,s$, so $f$ has the general structure of $f= f(g,s)$, defined over the $P$'s, $f= f(g(P),s(P))$. For example, cost $f$ may have an additive functional structure. Thus, (1) may be formulated as a linear or other functional combination of $g,s$:

$$P* = argmin_P \beta\, g(P) + \gamma\, s(P) \qquad (2)$$

The reals $\beta,\gamma \geq 0$ are the above mentioned strength parameters. They may be normalized such that $y = \beta - 1$.

If $P$ is chosen in a parameterization of overlap reference points, such as center points $x=x_j$, eq (2) can be further specialized into:

$$P* = \underset{x_1, x_2, \dots x_n}{argmin} \sum_{x_j}^N g(x) + \gamma s(x)$$

$$x_j \in [(x_S + n*h) - \delta, (x_S + n*h) + \delta] \qquad (3)$$

In this non-limiting example, $\beta=1$. The strength or weighting factors $\beta,\gamma$ define the trade-off between dose reduction and chance for stitching failures. Setting $y=0$ will result in planned-field of views $v_j$ selection ideal for dose reduction, while setting e.g. $\lambda = 1$ will result in a setting where the planned-field of views $v_j$ are selected in a way to obtain ideal stitching conditions for calculating the super-image M. The optimization above assumes that $s, g$ are in the range of $0, \dots ,1$, at least are non-negative. The variable $\delta$ defines the search range. The formulation above assumes that the initial height of the planned-field of views $v_j$ is adjusted according to the chosen midpoints. As the height of the planned-field of views $v_j$ is limited by the parallax error in case of parallel stitching, the search range can be further reduced to a set of technically feasible heights which would be an additional constraint to the formulation above. Furthermore, in some setups, the second or subsequent center-point $x_j+1$ will depend on the choice of the first or earlier one $x_j$ because the choice of $x_j$ may affect the height of the second planned-field of views $v_j$. In eq (2,3), the $P = \{x_j\}$ implicitly define the $v_j$'s.

One solution to solve Eqs. (1-3) may be to use an exhaustive search. This may be computationally expensive as it scales exponentially with the number of planned-field of views $v_j$. Alternatively, an iterative approach optimizing one point at a time may be used instead, although care must be taken not to miss the global minimum. One option around this is to include the height/size of the planned-field of views $v_j$ in the fixed contextual parameters u, thus restricting the search/solution space to recommended heights/size for the planned-field of views $v_j$, which will reduce computational cost for the search.

Optionally, with confinement of solution space as $\delta$ neighborhoods placed at multiples $n$ (natural number 1, 2 ... ) of $u=h$ (the pre-defined expected width of planned FOVs $v_j$ and hence of sub-images $m_j$). In the example of (3), the parametrization is judiciously chosen to run in 1D along one of the axes (in this X, along patient's longitudinal axis).

The cost function $f$ in Eqs. (1-3) is configured to favor regions for placement of the $x_j$'s (or definition of the partition more generally) with low self-similarity (so strong heterogeny) over homogenous regions (high self-similarity), and, at the same time, take into account radiation sensitivity at the particular locations, with a tendency to place overlaps over regions with lower radiation sensitivity. The constraints or objectives expressed by maps $g$ and $s$ are in a sense opposed, and the setup at Eq. (x) aims that finding an equilibrium solution $P*$ in taking into account both objectives, $g$ and $s$. A strength parameter $\beta$ (a type of regularization) can be set by user or may be preset by manufacturer or systems engineer via suitable system interface, via user interface UI or other. The strength parameter defines which objective $g, s$ is to dominate over the other. Setting $p =0.5$ will ensure equal weighting of both objectives.

If the size of the overlap, such as $\delta$, is not pre-defined ($\delta \notin$ u) but forms part of the solution variables over which the optimization eq (1-3) is run, there is tendency in the optimization setup to favor smaller (not to large) overlap sizes. Thus, the effect of the self-similarity cost, which tends to favor larger overlaps, is mitigated and counteracted by the radiation sensitivity cost. If the overlap is allowed too large, it is bound to spill over into radiation sensitive regions, thus incurring too much of a cost as per the radiation sensitivity map. The proposed setup allows taking entrance dose depending on dose absorption of different anatomical regions into account.

For solving/finding solutions to Eqs. (1-3) above, the partitioner PR may use, instead of the above mentioned searches, iterative-analytical algorithmic approaches, generally known as such in scientific computing/numerical analysis field , such as gradient based methods, conjugate gradients, stochastic gradient, Nelder-Mead, Newton-Raphson, or others.

The above mentioned imaging geometry adjuster IGA is optional. This is because, instead of the partition data being geometrical, defining data points P in the planned projection domain, the above described partitioning optimization may directly operate in (that is, optimize over) the space $G$ of imaging geometry settings $gj \in G$. Thus, the partitioning is over space G instead, in terms of angels, lengths etc., quantities that determine imaging geometry settings. However, because each imaging geometry $gj$ is linked one-to-one to a specific (planned) FOV $vj$, space G is nothing but a reparameterization of the planned projection domain. Thus, the above described operation of optimizing Eqs. (1-3) over domain $\pi$ in terms of geometrical descriptors P of the $vj$'s is without loss of generality, and thus in no way limiting. Thus, the proposed partitioning via Eqs. (1-3) or similar over domain $\pi$, specifically includes herein all other counterpart optimizations over $G$, or any other parameterization in relation to $\pi$.

In all of the above Eq (1-3), instead of using radiation sensitivity and structure self-similarities measures $g, s$. dual formulations are also envisaged where radiation in-sensitivity map and structure self-dissimilarity or variability is used instead. In such case, optimization Eq (1-3) may be reframed as a maximization of $f$, now being framed as a utility function.

Reference is now made to Fig. 5 in order to illustrate the merits of the proposed scheme. Fig. 5A is a qualitative representation of the cost function of a standard image stitching algorithm that may suffer from multiple local minima which may make robust definition of partition (placement of overlap, etc.) difficult, if not impossible. For example, as illustrated, there may be two local minima close to each other. Due to noise or parallax errors, the wrong local minimum might be chosen. Which leads to an inaccurate stitching result or complete failure of the stitching process.

In the proposed scheme, and, as illustrated in Fig. 5B, the cost function /here is better behaved, may include fewer, such as a single, local minimum of the cost function, or at least the global minimum of the cost function tends to be more pronounced in the proposed setup. This makes for more robust placement/partitioning, thanks to the consideration of the image structure similarity, and, optionally, but preferably, in conjunction with the radiation sensitivity.

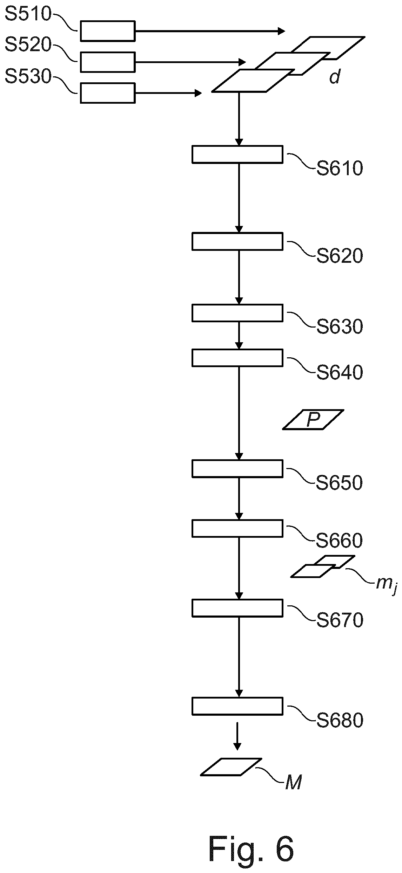

Reference is now made to Fig. 6, which shows a flow chart of the proposed method of facilitating obtaining sub-images for combination into a super-image, where the detector of the acquiring imaging apparatus is such that it is smaller than the region of interest to be imaged. The super-image affords a panoramic view of the ROI at once, or in parts, as needed.

Whilst the below steps of the method may be understood as a teaching in its own right, the below method can also be understood as a way of putting the above-mentioned facilitating system FS into effect.

Initially, operation of the method is based on input data $d$. Such input data $d$ may include a spatial range of the planned projection domain of the imager in the examination region of the imager in which the current patient resides. It is this range of region of the planned projection domain that is to be partitioned into planned FOVs $vj$, which can then be used to acquire the sub-images from which the super-image can then be combined. The partition is such that at least some or all of any two adjacent (neighboring) planned FOVs vj ought to overlap, subject to certain objectives $g, s$, as mentioned earlier. Finding such a partitioning is an objective of the proposed method. Such range data may be captured and supplied through user input data via user interface at step S510, or in automated fashion or in whichever way.

In addition, the input data d may include the spatial context data, such as the radiation sensitivity data map s and/or the structure self-similarity map g, as introduced above. Such data represents the said objectives. Each such data $g,s$ may be provided in a prior preparation step S520, S530 such as by medical modelling, using medical knowledge, atlases, etc. This data $g$ or $s$ may be generic at this point, or may be already specific to the given patient, as the case may be.

At step S610 such data $d$ is received, not necessarily all of range, and maps $g$ and $s$ at the same time.

If the spatial data $g,s$ is not already patient specific (is not already personalized), it is registered or interpolated or otherwise adapted/personalized at step S620 onto patient specific spatial measurements $\varphi$. If there already is native registration, etc., of the data for some reason, this step may be obsolete and not needed.

The step of acquiring such patient specific spatial measurements $\varphi$ may include operating the mentioned non-ionizing sensor SGS, or using the imager itself in a surview scan mode. Thus, the patient specific spatial measurements $\varphi$ may include spatial data, such as imagery (optical, depth, IR, LIDAR scan, etc.) of the patient PAT in the examination region. The registration results in the re-mapping of generic values of $g,s$ into points $p$ in the planned projection domain, $p\text{->}g(p), s(p)$. A forward projection onto planned projection domain $\pi$ may be done across generic model $g,s$ which may be 3D models.

Thus, registration step S620 may involve registration of standard data onto the actual measurement, preferably non-ionizing, of the patient to be imaged. Because of this registration, initial standard maps $g,s$ are personalized to patient as in the examination region of the given imaging equipment. The previous standard data is mapped onto the grid points of the planned projection domain for the imaging apparatus to be used. Thus, the registration assigns to each grid point $p$ in the plane the respective radiation sensitive value $s(p)$ and/or a structure similarity measure $g(p)$, as needed. The registration may include interpolation as the native, original grid size of $g,s$ may not match the grid system of planned projection domain $\pi$.

For example, the planned projection domain $\pi$ may include all positions of the tabletop of support PS on which patient lies, as this roughly corresponds to (such as is in a known spatial relation to) the surface in which the detector can be moved. The detector may be movably held in a tracked or other gantry under the table PS and can be thus slid into position. However, the particular nature of the planned projection domain will depend on the exact type of imaging setup, as this may imply certain constrains on the planned projection domain. For example, in chest-X ray with patient standing between source XS and detector XD (with detector XD behind patient's back for example) the planned projection domain $\pi$ may be thought of as the imaginary vertical plane behind the patient, in which the detector can be moved. Some of these movements may be "virtual" detector movements as it is instead, or in addition, the source XS that is moved, although with small-sized detectors as mainly envisaged herein it is more useful to have the detector move in imaging geometry adjustment. In some cases, both detector and source are moved, such as in C-arm systems.

At step S630 the partition $P$ is computed, based preferably on an optimization setup, driven by cost function $f$, such as in Eqs. (1-3) above. The cost function $f$ may be based on the above-mentioned maps $g,s$. The optimization of cost function is to minimize the cost function or at least improve cost. The cost is in terms of radiation sensitivity and/or the structure self-similarity, thus seeking out placement of partition borders at grid points associable with higher variability and over tissue of lower radiation sensitivity. This allows making the stitching algorithm more robust as regions with expected homogeneous distribution tend to be avoided as such regions provide no or few clues as to how to fit adjacent sub-images together, later, post-acquisition.

The partition (data) $P$ may then be made available at step S640. The partition data P via its elements (which may be 1D or 2D) defines regions in domain $\pi$, which region are the planned FOVs vj.

Such partition data $P$ may be used at step S650, for example to adjust the imaging geometry respectively so that to each partition element, say $xj$, a respective imaging geometry setting $gj$ is provided that is capable of instantiating its associated $vj$. There may be plural such settings $gj$, at least one for each $vj$.

At step S660 a sequence of sub-images $mj$ is acquired at the respective geometry settings $gj$, each of a different field of view as per the $vj$ of the partition data P. Thus, the planned (potential) FOVs $vj$ are now actual FOVs at which the sub-images are acquired.

Thus, once a given adjustment $gj$ is done, one or more exposures via source XS are triggered to so acquire the sub-images $mj$. The imaging geometry $gj$ is then re-adjusted into $gj+1$, and the next sub-image $mj+1$ is then acquired, and so on in sequence, until the whole of the region of interest is covered, which is implicit because of the partitioning.

At step S670 the so acquired sub-images $\{mj\}$ are then made available. For Example, some or all of the $mj$'s may be combined at step S680 at their respective border portions into the sought target or super-image $M$.

In some embodiments, the combining may include using, within the respective overlap, only image information (pixel values) from one of the sub-image pairs that are joined up at the overlap, instead of image information from the other sub-image of the pair $mj,mj+1$. In other embodiments, image information from both sub-images is used to fill in overlap pixels, such as by blending, averaging, or in whichever way. Such a combining from both images $mj,mj+1$ by blending etc., may allow to offset for some errors or inaccuracies that may have been incurred when computing partition $P$, e.g., by solving eqs(1-3). The combining may also be referred to herein as stitching. Combing includes any operation or merging of neighboring sub-images that comprise a specified overlap. The manner in which the information comprised in the overlap may be used may be different for different combining/stitching algorithms. In particular, such combing may use (normalized) cross-correlation, or the sum of absolute differences between the image intensity signals, etc. based on information as in the one or more overlap.

The super-image $M$ or parts thereof may then be provided for visualization on the display device or may be otherwise processed, stored as needed.

The sensor (e.g., camera SGS) through which the patient specific spatial measurements $\varphi$ are obtained may include optical imagery or infra-red imagery depth sensing camera imagery, color or grey value imagery with a depth channel or any other. The camera SGS may be integrated into the imaging apparatus IA or may be elsewhere mounted in the exam room to acquire the patient specific spatial measurements $\varphi$ for a patient in the examination region, of whom the super-image M is to be obtained. The range, as mentioned for "kick-starting" the above method, may be specifically prescribed by the user, but can also be obtained, based on the said measurements $\varphi$ of the patient in the examination region. Segmentation algorithms, powered by artificial intelligence or analytical, may be used to (semi-automate this data provision step.

It should be understood that the parameterization, over which the optimization of the cost function is performed, in terms of center points of the overlaps is merely one example which, however, has proven to yield good results. Other parametrizations in terms of other reference points to describe the pre-acquisition planned sub field of views as portions of the planned projection plane $\pi$ are also envisaged herein in embodiments, and such can be used instead or in addition. In the parameter relation used, the overlap may be defined implicitly or explicitly, as in the above example. The parametrization may include more than one single aspect, such as the center reference points of the overlaps. For example, further aspects may be parameterized, and made part of the solution space, such as the number of overlaps, the size of the planned field of views etc., as needed.

As can be understood from the above, the proposed method up to step S650 is operable pre-acquisition, whilst the remaining steps are done post-acquisition. In parts, the proposed method is thus pre-acquisition, a planning method, for planning the acquisition of sub-images for combination into the super-image for the region of interest that is thought to be larger than the size of the detector to be used.

The components of the facilitator FS may be implemented as one or more software modules, run on one or more general-purpose processing units such as a workstation associated with the imager IA, or on a server computer associated with a group of imagers.

Alternatively, some or all components of the facilitator FS may be arranged in hardware such as a suitably programmed microcontroller or microprocessor, such an FPGA (field-programmable-gate-array) or as a hardwired IC chip, an application specific integrated circuit (ASIC), integrated into the imaging system IA. In a further embodiment still, the facilitator FS may be implemented in both, partly in software and partly in hardware.

The different components of the facilitator FS may be implemented on a single data processing unit. Alternatively, some or more components are implemented on different processing units, possibly remotely arranged in a distributed architecture and connectable in a suitable communication network such as in a cloud setting or client-server setup, etc.

One or more features described herein can be configured or implemented as or with circuitry. Circuitry may include discrete and/or integrated circuitry, a system-on-a-chip (SoC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it, which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

**Claims**

1.  A system (SF) for facilitating obtaining sub-images ($mj$) of a region of interest, ROI, (R) for combining into a target image (M) of the ROI (R), the said ROI (R) being situatable in an examination region (ER) of an X-ray imaging apparatus (IA), wherein the system is to receive, at an input interface (IN), input data including data ($d$) indicative of the ROI, and the system capable of computing, prior to X-ray exposure, based on the input data, a plurality of sub-FOVs, wherein the plurality of sub-FOVs in combination are capable to cover the ROI.

2.  System of claim 1, the system capable of so computing based on a partitioner (PR), wherein partitioner (PR) is operable to compute, based on the input data, a partition ($P$) of at least a portion of a planned projection domain ($\pi$) in relation to the imaging apparatus into planned sub-field-of-views, FOV, (vj) at which the sub-images are acquirable by the X-ray detector at different imaging geometries ($gj$).

3.  System of claim 1 or 2, comprising an output interface (OUT) for passing on a specification of the partition to control circuitry (CC) of the imaging apparatus (IA) for processing and based on the processing, to causing the imaging apparatus (IA) to acquire the sub-images ($mj$) at the sub-FOVs (vj).

4.  System of claim 3, wherein the control circuitry (CC) to include, or capable of interfacing with, an imaging geometry determiner (IGA) capable of determining imaging geometry settings ($gj$) that enable the imaging apparatus to acquire said sub-imagery at the sub-FOVs (vj).

5.  System of claim 3 or 4, comprising an image combiner ($\Sigma$) capable of combining the sub-images, once acquired, into the target image (M).

6.  System of any one of the preceding claims, wherein there is at least one overlap ($\lambda$) of adjacent sub-FOVs (vj, $v_{j+1}$).

7.  System of claim 6, wherein the partition (P) is definable in terms of location of the said at least one overlap ($\lambda$) in the projection domain ($\pi$), given an overlap size and/or size of the sub-FOVs.

8.  System of any one of the preceding claims, wherein the input data includes specification of any one or more of:

    i) a respective size ($h$) of the sub-FOVs,
    ii) an indication of a size of the, or a, at least one overlap ($\lambda$) of any adjacent sub-FOVs, and
    iii) spatial data indicative of spatial distribution of any one of more of a) local radiation sensitivity of the ROI, and b) local structural data within the ROI.

9.  System of any one of the preceding claims, wherein operation of the partitioner (PR) is driven by an objective function ($f$) in an optimization setup.

10. System of any one of the preceding claims, wherein the objective function ($f$) is configured to respond to any one or both of: a) distribution of local radiation sensitivities of the ROI, and b) distribution of local structural data within the ROI.

11. A medical imaging arrangement (MIR), comprising at least a part of the system of any one of the preceding claims, and further comprising any one or more of: i) the imaging apparatus, ii) one or more memories (MEM) on which is stored at least part of the input data, iii) the said sensor (SGS).

12. A computer-implemented method of facilitating obtaining sub-images (mj) of a region of interest, ROI, (R) for combining into a target image (M) of the ROI (R), the said ROI (R) being situatable in an examination region (ER) of an X-ray imaging apparatus (IA), the method comprising:
    receiving (S610) input data including:

data (d) indictive of the ROI; and

computing (S630), prior to X-ray exposure, based on the input data, a plurality of sub-FOVs, wherein the plurality of sub-FOVs in combination are capable to cover the ROI.

13. Method of generating the said spatial or prior data as per any one of claim 8-10.

14. A computer program element, which, when being executed by at least one processing system (PS, FS), is adapted to cause the processing system (PS, FS) to perform the method as per any one of claims 12 or 13.

15. At least one computer readable medium having stored thereon the program element of claim 14.

Fig. 1

Fig. 2

Fig. 3

Fig. 4

A)

B)

Fig. 5

S510

S520

S530

S610

S620

S630

S640

$P$

S650

S660

$m_j$

S670

S680

$d$

$M$

# Fig. 6

Europäisches
Patentamt
European
Patent Office
Office européen
des brevets

# EUROPEAN SEARCH REPORT

**Application Number**

EP 24 22 0768

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/247081 A1 (HALSMER MATTHEW A [US] ET AL) 9 December 2004 (2004-12-09) * paragraphs [0006] - [0074]; figures 1-7 * | 1-15 | INV. A61B6/00 |
| X | US 2014/016755 A1 (BEHIELS GERT [BE]) 16 January 2014 (2014-01-16) * paragraphs [0010] - [0019]; claims 1-5; figures 1-4 * | 1-15 | |
| X | US 2020/237332 A1 (WANG DEJUN [CN] ET AL) 30 July 2020 (2020-07-30) * paragraphs [0012] - [0043]; figures 1-6 * | 1-7, 11-15 | |
| X | US 2017/055925 A1 (LEE HO JUN [KR] ET AL) 2 March 2017 (2017-03-02) * paragraphs [0277] - [0291]; figures 27-30 * | 1-7, 11-15 | |

**TECHNICAL FIELDS SEARCHED (IPC)**

A61B

The present search report has been drawn up for all claims

| Place of search | Date of completion of the search | Examiner |
|---|---|---|
| Munich | 14 May 2025 | Daoukou, Eleni |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 22 0768

This annex lists the patent family members relating to the patent documents cited in the above-mentioned European search report.
The members are as contained in the European Patent Office EDP file on
The European Patent Office is in no way liable for these particulars which are merely given for the purpose of information.

14-05-2025

| Patent document cited in search report | Publication date | Patent family member(s) | Publication date |
|---|---|---|---|
| US 2004247081 A1 | 09-12-2004 | EP 1484016 A1 | 08-12-2004 |
| | | EP 1484017 A1 | 08-12-2004 |
| | | JP 4458936 B2 | 28-04-2010 |
| | | JP 4545490 B2 | 15-09-2010 |
| | | JP 2004358254 A | 24-12-2004 |
| | | JP 2004358255 A | 24-12-2004 |
| | | US 2004247081 A1 | 09-12-2004 |
| | | US 2005169427 A1 | 04-08-2005 |
| US 2014016755 A1 | 16-01-2014 | BR 112013024519 A2 | 24-09-2019 |
| | | CN 103458788 A | 18-12-2013 |
| | | EP 2508132 A1 | 10-10-2012 |
| | | US 2014016755 A1 | 16-01-2014 |
| | | WO 2012136520 A1 | 11-10-2012 |
| US 2020237332 A1 | 30-07-2020 | CN 111466932 A | 31-07-2020 |
| | | US 2020237332 A1 | 30-07-2020 |
| US 2017055925 A1 | 02-03-2017 | EP 3155969 A1 | 19-04-2017 |
| | | US 2017055925 A1 | 02-03-2017 |
| | | US 2020330062 A1 | 22-10-2020 |
| | | US 2022346736 A1 | 03-11-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Recommendations of the International Commission on Radiological Protection. *ICRP Publication 103, Ann. ICRP*, 2007, vol. 37 (2-4), 2007